# EUROPÄISCHE PATENTSCHRIFT

(11) **EP 0 789 015 B1**
(45) Veröffentlichungstag und Bekanntmachung des Hinweises auf die Patenterteilung: **29.12.1999**
(21) Anmeldenummer: 97101595.3
(22) Anmeldetag: 03.02.1997
(51) Int. Cl.: C07C 227/08, C07C 229/56, C07C 249/02

(54) **Verfahren zur Herstellung von N-Carboxymethylenanthranilsäure-Estern**
Process for the preparation of N-carboxymethylanthranilic acid esters
Procédé pour la préparation d'esters de l'acide N-carboxyméthylanthranilique

(30) Priorität: 09.02.1996 DE 19604707
(43) Veröffentlichungstag der Anmeldung: 13.08.1997
(73) Patentinhaber: Clariant GmbH, 65929 Frankfurt am Main (DE)
(72) Erfinder: Papenfuhs, Theodor, Dr., 60433 Frankfurt (DE); Dierdorf, Andreas, Dr., 60529 Frankfurt (DE); Krause, Stefan, Dr., 65843 Sulzbach/Ts (DE); Neumann-Grimm, Doris, Dr., 60388 Frankfurt (DE)

(56) Entgegenhaltungen:
- WO-A-95/18093
- JOURNAL OF HETEROCYCLIC CHEMISTRY, Bd. 25, 1988, PROVO US, Seiten 1831-1835, XP000670083 ELISABETTA BORRIONE ET AL.: "Synthesis and cycloaddition reactions of ethyl glyoxylate imines..."

## Beschreibung

Die vorliegende Erfindung betrifft ein Verfahren zur Herstellung von N-Carboxymethylenanthranilsäure-Estern.

N-Carboxymethylenanthranilsäure-Ester, insbesondere N-Alkoxycarbonylmethylenanthranilsäuren, besitzen beispielsweise bei der Herstellung von Indigo technische Bedeutung.

Gemäß Ullmann's Encylopedia of Industrial Chemistry, Vol. A14, Seiten 150 bis 151 läßt sich N-Carboxymethylenanthranilsäure durch Umsetzung von Anthranilsäure mit Chloressigsäure herstellen. Die N-Carboxymethylenanthranilsäure kann anschließend unter Verwendung von Schwefelsäure als Katalysator mit Ethanol verestert werden. Nachteilig an dieser Synthese ist die Verwendung von Chloressigsäure, die zwangsweise zu chlorhaltigen Abfallprodukten führt. Chlorhaltige Abfallprodukte sind jedoch aus Gründen des Umweltschutzes unerwünscht und sollten daher vermieden werden. Von Nachteil ist ferner der Gebrauch von Schwefelsäure, da sich säurehaltige Rückstände bilden, die ebenso wie die chlorhaltigen Abfallprodukte Probleme bei der Entsorgung bereiten.

Borrione, Prato, Scorrano, Stivanell und Lucchini beschreiben in J. Heterocyclic Chem., 25, 1831 bis 1835 (1988) die Synthese von Glyoxylsäureethylester-Iminen und einige ihrer Cycloadditionsreaktionen. Während sich Aniline mit elektronenschiebenden Gruppen bei 25°C in Toluol oder Dichlormethan problemlos mit Glyoxylsäureethylester zu den entsprechenden Iminen umsetzen lassen, reagieren Aniline mit elektronenziehenden Gruppen träge und erfordern schärfere Reaktionsbedingungen.

Als Aniline mit elektronenschiebenden Gruppen sind Anilin (2a), p-Toluidin (2b) und p-Methoxyanilin (2c) und als Aniline mit elektronenziehenden Gruppen sind p-Chloranilin (2d), p-Nitroanilin (2e) und o-Nitroanilin untersucht werden. Die Umsetzung der Aniline erfolgt mit Glyoxylsäureethylester-Hydrat in Gegenwart von wasserfreiem Natriumsulfat in Toluol (siehe auch Seite 1834, rechte Spalte: Experimental). Die Aniline mit elektronenziehenden Gruppen werden wegen ihres reaktionsträgen Verhaltens bei 110°C umgesetzt und liefern, belegt durch NMR-Überwachung, komplexe Reaktionsgemische. Es wird gefolgert, daß die komplexen Reaktionsmischungen, die durch Umsetzung equimolekularer Mengen Glyoxylsäureethylester und Anilin mit elektronenziehenden Gruppen in Toluol erhalten werden, aus freiem Imin (Anil) und aus Additionsprodukten, die durch Addition von Wasser oder nichtumgesetzten Anilin an die Kohlenstoff-Stickstoff Doppelbindung gebildet werden, bestehen (siehe Seite 1831, linke Spalte unten und rechte Spalte oben und rechte Spalte, vorletzter Abschnitt).

Die Umsetzung von Glyoxylsäureester mit Anilinen liefert sowohl im Falle der Aniline mit elektronenschiebenden Gruppen als auch im Falle der Aniline mit elektronenziehenden Gruppen Imine, die recht instabil sind und sich nicht reinigen lassen. Ein Versuch, die Imine durch Chromatographie oder Destillation zu reinigen, führt zu weitgehender Zersetzung (siehe Seite 1834, Experimental General Procedure).

In J. Heterocyclic Chem., 25, 1831 bis 1835 (1988) werden einige Cycloadditionen unter Verwendung der Glyoxylsäureethylester-Imine, nicht jedoch die Herstellung von N-Carboxymethylenanthranilsäure-Estern beschrieben.

WO 95/18093 betrifft ein Verfahren zur Herstellung von N-substituierten Glycinsäuren oder Glycinestern und Verwendung des Verfahrens zur Herstellung von Indigo. Man setzt ein Amin mit einem Glyoxylsäureesterhalbacetal oder Glyoxylsäurehalbacetal um und hydriert das dabei entstehende Zwischenprodukt. Als Lösungsmittel wird der Alkohol eingesetzt, der den gleichen Alkylrest wie der Alkylteil des jeweils verwendeten Halbacetals aufweist. Die Umsetzung von Anthranilsäuremethylester mit Glyoxylsäuremethylestermethylhemiacetal ist in Beispiel 3 (siehe Seite 9; Tabelle 1) beschrieben. Sowohl der Anthranilsäuremethylester als auch das Glyoxylsäuremethylestermethylhemiacetal werden - gelöst in einem großen Methanol-Überschuß - eingesetzt. Der Fortgang der Reaktion wird mittels Dünnschichtchromatographie verfolgt. Nach beendeter Reaktion wird die resultierende Reaktionslösung in einem separaten Schritt in Gegenwart eines Nickelkatalysators hydriert. Nachteilig ist die Verwendung von Methanol als Lösungsmittel, da dieses als protisches Lösungsmittel bei einer Weiterverarbeitung des erhaltenen N-Carbomethoxyphenylglycinsäuremethylesters stört und deshalb abgetrennt werden muß. Zudem ist die Verwendung von Glyoxylsäuremethylestermethylhemiacetal mit einem zusätzlichen Aufwand verbunden, da dieses Hemiacetal üblicherweise durch Umsetzung von Glyoxylsäure mit Methanol hergestellt wird. Von Nachteil ist auch, daß im Verlauf der Umsetzung aus dem Hemiacetal zwangsweise Methanol abgespalten wird, das ebenfalls bei einer Weiterverarbeitung, beispielsweise bei einer Umsetzung mit Phosgen, stört und deshalb zu entfernen ist.

Es besteht daher ein Bedarf, ein Verfahren bereitzustellen, das die vorstehend genannten Nachteile vermeidet und sich zudem auf einfache Art und Weise realisieren läßt, ohne großen technischen Aufwand zu erfordern. Ferner soll das Wertprodukt in guter Ausbeute und zugleich hoher Reinheit gewonnen werden. Darüber hinaus sollen Abfallprodukte soweit als möglich vermieden werden, insbesondere solche die unter dem Gesichtspunkt des Umweltschutzes bedenklich sind.

Gelöst wird diese Aufgabe durch ein Verfahren zur Herstellung von N-Carboxymethylenanthranilsäure-Estern der Formel (3)

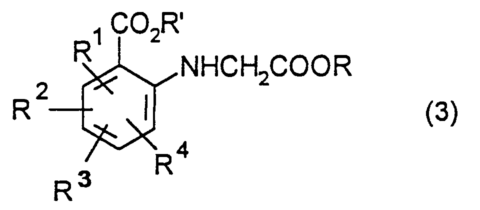

Es ist dadurch gekennzeichnet, daß man eine Verbindung der Formel (1) 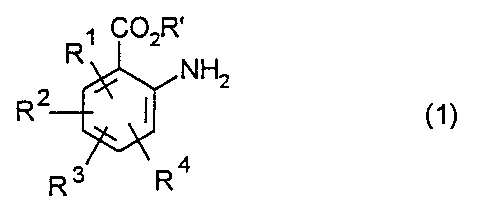 worin R' für Wasserstoff oder einen geradkettigen oder verzweigten Alkylrest mit 1 bis 4 Kohlenstoffatomen steht, R¹, R², R³, R⁴ gleich oder verschieden sind und unabhängig voneinander für Wasserstoff, Halogen, NO₂, einen geradkettigen oder verzweigten Alkyl-, Alkoxy- oder halogenierten Alkyl-Rest mit jeweils 1 bis 6 Kohlenstoffatomen stehen, mit einem Glyoxylsäureester der Formel (2)

OHC-COOR (2)

worin R für einen unsubstituierten oder einen durch Halogen, eine Alkyl- oder Alkoxygruppe mit jeweils 1 bis 4 Kohlenstoffatomen einfach oder mehrfach substituierten geradkettigen oder verzweigten Alkylrest mit 1 bis 20 Kohlenstoffatomen, Phenylrest oder Benzylrest steht, in Anwesenheit eines Lösungsmittels ausgenommen Alkohole bei 20 bis 200°C umsetzt und das Reaktionsprodukt mit Wasserstoff in Anwesenheit eines Nickel, Kobalt, Platin, Palladium, Rhodium, Ruthenium oder Verbindungen dieser Metalle enthaltenden Hydrierkatalysators bei einer Temperatur von 0 bis 200°C und einem Druck von 1 bis 150 bar reduziert.

Die Umsetzung der Verbindung der Formel (1) mit dem Glyoxylsäureester der Formel (2) verläuft über die Bildung eines Imins. Bei den Verbindungen der Formel (1) handelt es sich um Aniline die eine CO₂R' Gruppe besitzen, worin R' für Wasserstoff oder einen geradkettigen oder verzweigten Alkylrest mit 1 bis 4 Kohlenstoffatomen steht. Unabhängig von der Bedeutung von R' stellt die CO₂R' Gruppe eine elektronenziehende Gruppe dar. Sowohl die Carbonsäuregruppe (R' = H) als auch die Carbonsäureestergruppe (R' = Alkyl mit 1 bis 4 Kohlenstoffatomen) besitzt nicht nur einen -J Effekt sondern auch einen -M Effekt. Diese beiden Effekte belegen, daß es sich bei der CO₂R' Gruppe in Verbindung (1) um eine elektronenziehende Gruppe handelt.

Im Hinblick auf die Erläuterung von J. Heterocycl. Chem., 28, 1831 bis 1835 (1988) ist es als überraschend anzusehen, daß Aniline mit einem elektronenziehenden Rest CO₂-R' sich trotz ihrer geringen Reaktivität und trotz der geringen Stabilität und der Neigung ihrer mit Glyoxylsäureester gebildeten Imine, mit Wasser und nicht umgesetztem Anilin (Verbindung der Formel (1)) zu reagieren, mittels des erfindungsgemäßen Verfahren in die entsprechenden N-Carboxymethylenanthranilsäure-Ester überführen lassen.

Es war nicht zu erwarten, daß die Umsetzung relativ glatt abläuft und keine übermäßig scharfen Reaktionsbedingungen erfordert und daß sich zudem die Bildung unerwünschter Nebenprodukte in Grenzen hält.
Es ist ein Vorteil, daß auf den Einsatz eines Hemiacetals und den Gebrauch eines Alkohols als Lösungsmittel verzichtet werden kann, so daß in die Weiterverarbeitung eine alkoholfreie Lösung des N-Carboxymethylenanthranilsäure-Esters gelangt. Dadurch werden unerwünschte Nebenreaktionen, die auf den Alkohol zurückgehen, vermieden.

Man setzt üblicherweise eine Verbindung der Formel (1), worin R' für Wasserstoff, Methyl oder Ethyl, insbesondere für Wasserstoff oder Methyl, bevorzugt für Wasserstoff steht, ein.

Man kann in die Umsetzung mit gutem Erfolg eine Verbindung der Formel (1), worin R¹, R², R³, R⁴ gleich oder verschieden sind und unabhängig voneinander Wasserstoff, Fluor, Chlor, einen Alkyl-, Alkoxy- oder chlor- oder fluorsubstituierten Alkyl-Rest mit jeweils 1 bis 4 Kohlenstoffatomen stehen, insbesondere R¹, R², R³, R⁴ gleich oder verschieden sind und unabhängig voneinander für Wasserstoff, Fluor, Chlor, Methyl oder Ethyl stehen, einsetzen. Von Interesse sind Verbindungen der Formel (1), worin zwei oder drei der Reste insbesondere drei der Reste R¹, R², R³, R⁴ für Wasserstoff stehen. Als gängige Verbindungen der Formel (1) seien, ohne Anspruch auf Vollständigkeit zu erheben Anthranilsäure, Anthranilsäuremethylester, Anthranilsäureethylester, 4-Chloranthranilsäure, 4-Chloranthranilsäuremethylester, 4-Chloranthranilsäureethylester, insbesondere 4-Chloranthranilsäure genannt.

Man setzt einen Glyoxylsäureester der Formel OHC-COOR (2) ein, worin R die eingangs erwähnte Bedeutung hat, insbesondere R für einen Alkylrest mit 1 bis 6 Kohlenstoffatomen, bevorzugt für Methyl oder Ethyl, besonders bevorzugt für Ethyl steht.

Die Umsetzung der Verbindung der Formel (1) mit der Verbindung der Formel (2) läuft in Anwesenheit eines Lösungsmittels ab. Als Lösungsmittel kann man einen aromatischen Kohlenwasserstoff, einen chlorierten aliphatischen oder aromatischen Kohlenwasserstoff, einen Alkylester einer aliphatischen Carbonsäure mit 1 bis 4 Kohlenstoffatomen, ein Carbonsäureamid, eine N-enthaltende heterocyclische Verbindung, einen Ether oder eine aliphatische Carbonsäure mit 1 bis 4 Kohlenstoffatomen oder ein Gemisch derselben, insbesondere einen aromatischen Kohlenwasserstoff, einen chlorierten aromatischen Kohlenwasserstoff, einen Carbonsäureester oder ein Gemisch derselben verwenden.

Als Lösungsmittel eignen sich beispielsweise Toluol, o-Xylol, m-Xylol, p-Xylol, ein Gemisch isomerer Xylole, Ethylbenzol, Mesitylen, Chloroform, Dichlormethan, Chlorbenzol, Dichlorbenzol, Ethylacetat, Butylacetat, N,N-Dimethylacetamid, N,N-Diethylacetamid, N-Methylpyrrolidon, Tetrahydrofuran, Dioxan, Diisopropylether, Di-n-butylether, Essigsäure, Propionsäure oder ein Gemisch derselben, insbesondere Toluol, o-Xylol, m-Xylol, p-Xylol, ein Gemisch isomerer Xylole, Ethylbenzol.

In vielen Fällen haben sich Toluol, o-Xylol, m-Xylol, p-Xylol, ein Gemisch isomerer Benzole als Lösungsmittel bewährt.

Wie eingangs bereits erwähnt, führt man die Umsetzung der Verbindung der Formel (1) mit der Verbindung der Formel (2) bei 0 bis 200°C durch. In einer Vielzahl von Fällen hat es sich bewährt, die Umsetzung bei 10 bis 100, insbesondere 15 bis 50°C ablaufen zu lassen.
Während vergleichsweise reaktive Verbindungen der Formel (1) bei niedrigen Temperaturen, beispielsweise bei 10 bis 40°C zur Umsetzung gelangen, erfordert die Umsetzung vergleichsweise reaktionsträger Verbindungen der Formel (1) höhere Temperaturen, beispielsweise 60°C und darüber. Die jeweils anzuwendende Reaktionstemperatur ist in gewissem Umfang an die jeweils zum Einsatz gelangende Verbindung der Formel (1) anzupassen, um eine günstige Durchführung der Reaktion zu gewährleisten.

Wegen ihrer überraschend starken Neigung zur Decarboxylierung sind die Verbindungen der Formel (1), worin R' = H (also die Anthranilsäuren) unter milden Bedingungen umzusetzen. Daher empfiehlt es sich, sie bei 0 bis 50, insbesondere 10 bis 40, bevorzugt 15 bis 35°C umzusetzen.

Die Anthranilsäureester - also die Verbindungen der Formel (1), worin R' für einen geradkettigen oder verzweigten Alkylrest mit 1 bis 4 Kohlenstoffatomen steht - zeigen geringe Tendenz zur Decarboxylierung. Sie lassen sich daher auch bei höheren Temperaturen, beispielsweise bei 50°C und darüber, umsetzen. In einer Reihe von Fällen kann man sie unter den für Anthranilsäure genannten milden Bedingungen umsetzen.

Bei der Durchführung der Reaktion bei tiefen Temperaturen ( < 50°C) hat es sich bewährt, die Glyoxylsäureester-Lösung vor dem Einsatz einige Stunden auf > 100°C zu erhitzen, um polymere Bestandteile in monomere Glyoxylsäureester zu spalten.
Das erfindungsgemäße Verfahren verläuft in zwei Stufen. In der ersten Stufe entsteht das Additionsprodukt aus der Anthranilsäure respektive aus dem Anthranilsäureester der Formel (1) und dem Glyoxylsäureester (2). Es kann angenommen werden, daß sich das entsprechende Imin bildet und gleichzeitig Wasser abgespalten wird. Dieses Additionsprodukt wird in der zweiten Stufe mit Wasserstoff reduziert, wobei der entsprechende N-Carboxymethylenanthranilsäure-Ester der Formel (3) erzeugt wird.

Das Verfahren läßt sich bezüglich des auftretenden Reaktionswassers flexibel gestalten. Es ist nämlich möglich, auf eine Abtrennung des Reaktionswassers zu verzichten und es im Reaktionsgemisch zu belassen. In diesem Fall schließt sich die Reduktion des Additionsproduktes unmittelbar an die erste Stufe an, ohne das Reaktionswasser zuvor zu entfernen. Dadurch entfällt in vorteilhafter Weise ein separater Arbeitsschritt.

Es ist aber auch möglich, das aus der Umsetzung in der ersten Stufe resultierende Reaktionswasser zu entfernen. Üblicherweise trennt man das bei der Umsetzung gebildete Wasser mittels azeotroper Destillation, mittels eines wasserbindenden Mittels oder mittels Phasentrennung oder durch eine Kombination dieser Methoden ab.
Als wasserentziehendes Mittel eignen sich beispielsweise wasserfreies Natriumsulfat, Calciumchlorid oder ein Molekularsieb.

In einer Reihe von Fällen wird man für die Wasserabtrennung einer azeotropen Destillation oder einer Phasentrennung oder einer Kombination dieser beiden Methoden den Vorzug geben.
Häufig hat sich die azeotrope Destillation für die Wasserabtrennung als besonders geeignet erwiesen, insbesondere schon während der Umsetzung. Dabei ist es besonders einfach, das Wasser in dem Maße, wie es sich während der Umsetzung bildet, azeotrop abzudestillieren. In diesem Fall führt man die Umsetzung bei der Siedetemperatur des eingesetzten Lösungsmittels durch.

Man kann aber auch die Wasserabtrennung nach der in der zweiten Stufe durchgeführten Reduktion vornehmen, wobei man ebenso, wie vorstehend bei der Abtrennung des Wassers nach der ersten Stufe beschrieben, verfahren kann.

Man reduziert, wie eingangs bereits erwähnt, in Anwesenheit eines Hydrierkatalysators bei einer Temperatur von 0 bis 200°C und einem Druck von 1 bis 150 bar, insbesondere bei einer Temperatur von 10 bis 100°C, insbesondere 15 bis 50°C und einem Druck von 5 bis 60, insbesondere 15 bis 25 bar.

Da auch die Umsetzungsprodukte der Verbindungen der Formel (1), worin R' für H steht, (also die der Anthranilsäuren), mit dem Glyoxylsäureester der Formel (2) zur Decarboxylierung neigen, führt man die Hydrierung dieser Imine unter milden Bedingungen, beispielsweise bei 0 bis 50, insbesondere 10 bis 40, bevorzugt 15 bis 35°C, durch.

Da die Umsetzungsprodukte der Verbindungen der Formel (1), worin R' für einen geradkettigen oder verzweigten Alkylrest steht, (also die der Anthranilsäureester), mit dem Glyoxylsäureester der Formel (2) eine geringe Tendenz zur Decarboxylierung zeigen, lassen sie sich zwar auch unter den vorstehend genannten milden Bedingungen, aber auch bei höheren Temperaturen, beispielsweise bei 50°C und darüber hydrieren.

Als Hydrierkatalysatoren können üblicherweise solche verwendet werden, die bei der Herstellung von Aminen durch aminierende Hydrierung Einsatz finden. Diese Hydrierkatalysatoren enthalten üblicherweise Nickel, Kobalt, Platin, Palladium, Rhodium, Ruthenium oder Verbindungen dieser Metalle. In einer Reihe von Fällen setzt man als Hydrierkatalysator einen Nickel, Platin, Palladium oder Verbindungen dieser Metalle enthaltenden Katalysator ein.

Der Hydrierkatalysator kann geeignete Aktivatoren und Promotoren beinhalten.
Er kann trägerfrei oder auf einem Träger angeordnet sein.
Beispiele für trägerfreie Katalysatoren sind Raney-Nickel, Raney-Cobalt, Raney-Platin, Raney-Palladium.
Als Trägermaterial kommen Al₂O₃, Tonerde, Bimsstein, Kieselsäure, Kieselgel, SiO₂, Kieselgur oder Aktivkohle, insbesondere Al₂O₃, Kieselgur oder Aktivkohle in Betracht. Es lassen sich jedoch auch Mischungen dieser Stoffe als Trägermaterial verwenden. Das hydrieraktive Metall respektive die Metallverbindung kann durch Tränkung oder Fällung auf das Trägermaterial gebracht werden.
Gut geeignet sind Nickel enthaltende Trägerkatalysatoren auf Basis Tonerde, Kieselsäure, Kieselgel, SiO₂, Kieselgur oder Mischungen derselben, insbesondere Kieselsäure, Kieselgel, Kieselgur oder Mischungen derselben.
Geeignet sind ferner Trägerkatalysatoren auf Basis von Aktivkohle, insbesondere Platin oder Palladium enthaltende Aktivkohle-Katalysatoren.
Man kann den Katalysator in Form einer Suspension oder als Festbett angeordnet verwenden.

In einigen Fällen empfiehlt es sich, Sulfide der vorstehend genannten Metalle oder diese Sulfide enthaltende Katalysatoren einzusetzen oder zu den Hydrierkatalysatoren Schwefel oder Schwefel enthaltende Verbindungen, beispielsweise Alkalisulfit, Dimethylsulfoxid, oder andere die Aktivität des Katalysators in geeignetem Umfange vermindernde Stoffe, beispielsweise Chinolin, zuzusetzen. Es lassen sich hierfür auch sulfidierte oder sulfitierte Katalysatoren, insbesondere Platin oder Palladium enthaltende Katalysatoren, verwenden. Diese modifizierten Katalysatoren eignen sich besonders, das erfindungsgemäße Verfahren in einem Schritt, also die erste und zweite Stufe gleichzeitig, ablaufen zu lassen und bei der Verwendung halogenhaltiger Verbindungen der Formel (1), um eine unerwünschte Abspaltung des Halogens zu unterbinden.

Die Bedingungen für die Reduktion hängen auch von der Art des eingesetzten Katalysators ab. Während hochaktive Katalysatoren, beispielsweise Nickel enthaltende Katalysatoren, eine Hydrierung bei vergleichsweise niedrigen Temperaturen erlauben, kann der Einsatz weniger reaktiver Katalysatoren, beispielsweise schwefelmodifizierter Katalysatoren relativ höhere Reaktionstemperaturen erfordern.
Im Zweifelsfalle sollte die Art und Menge des Hydrierkatalysators vorab mittels einfacher Vorversuche, bei denen auch die Wahl des Lösungsmittels getroffen wird, ermittelt werden.

Eine besonders günstige Verfahrensvariante besteht darin, die Verbindung der Formel (1), die Verbindung der Formel (2), das Lösungsmittel, den Hydrierkatalysator und Wasserstoff zusammen vorzulegen und die Umsetzung und Reduktion zugleich durchzuführen. Die Wasserabtrennung erfolgt in diesem Fall nach Beendigung der Reaktion. Es ist aber auch möglich, das Wasser während der Reaktion auszukreisen. Vorteilhafterweise wird man jedoch die Wasserabtrennung erst nach Beendigung der Reaktion durchführen.

Man führt diese Verfahrensvariante (Eintopf-Verfahren) bei einer Temperatur von 0 bis 200, insbesondere 10 bis 100, bevorzugt 15 bis 50°C und einem Druck von 1 bis 150, insbesondere 5 bis 60, bevorzugt 15 bis 25 bar durch.

Während auch bei dieser Verfahrensvariante vergleichsweise reaktive Verbindungen der Formel (1) bei niedrigen Temperaturen, beispielsweise bei 10 bis 40°C zur Umsetzung gelangen, erfordert die Umsetzung vergleichsweise reaktionsträger Verbindungen der Formel (1) höhere Temperaturen. Gleiches gilt auch für die Umsetzungsprodukte der Verbindungen der Formel (1) mit dem Glyoxylsäureester der Formel (2).

Verbindungen der Formel (1), die zur Decarboxylierung neigen - beispielsweise wie zuvor erwähnt die Anthranilsäuren der Formel (1), worin R' für H steht - sowie leicht decarboxylierende Umsetzungsprodukte (Imine) unterzieht man bei milden Bedingungen der unter Wasserabspaltung ablaufenden Umsetzung und der Hydrierung der Umsetzungsprodukte (Imine). Die Verbindungen der Formel (1), worin R' für H steht, also die Anthranilsäuren setzt man beispielsweise bei 0 bis 50, insbesondere 10 bis 40, bevorzugt 15 bis 35°C um und führt die gleichzeitig ablaufende Hydrierung der Imine auch bei dieser Temperatur durch.

Anthranilsäureester, also Verbindungen der Formel (1), worin R' für einen geradkettigen oder verzweigten Alkylrest mit 1 bis 4 Kohlenstoffatomen steht, lassen sich aufgrund ihrer geringen Tendenz zur Decarboxylierung sowohl unter den vorstehend genannten milden Bedingungen als auch bei höheren Temperaturen, beispielsweise bei 50°C und drüber, umsetzen. Gleiches gilt auch für ihre Umsetzungsprodukte (Imine), die sich in einer Reihe von Fällen sowohl unter den vorstehend genannten milden Bedingungen als auch bei höheren Temperaturen, beispielsweise 50°C und darüber, hydrieren lassen.

Bei dieser Verfahrensvariante läuft die Wasserabspaltung und die Hydrierung zugleich ab und man kann die Wasserabspaltung und Hydrierung bei ein und derselben Temperatur ablaufen lassen.

Die nachfolgenden Beispiele belegen die Erfindung, ohne sie zu beschränken.

### Experimenteller Teil:

### Beispiel 1

### Herstellung von N-Ethoxycarbonylmethylenanthranilsäure (zweistufig)

Man legt 137 g (1 mol) Anthranilsäure, 204 g einer 50 %igen Lösung von Glyoxylsäureethylester in Toluol und 400 g Chloroform in einem mit einem Rückflußkühler und Wasserabscheider bestückten Glaskolben vor und erhitzt zum Rückfluß. Anfallendes Reaktionswasser wird während der Reaktion azeotrop abdestilliert und mittels des Wasserabscheiders ausgekreist. Das dabei anfallende Chloroform wird in die Reaktion zurückgeführt.
Der Fortgang der Umsetzung wird durch HPLC (High pressure liquid chromatography) analytisch überwacht. Die Reaktion ist nach etwa 4 Stunden abgeschlossen. Danach trennt man das Chloroform destillativ ab, überführt das Reaktionsgemisch in einen Hydrierautoklaven und setzt 5 g eines Palladium-Aktivkohlekatalysators (10 Gew.-% Palladium) zu.

Anschließend hydriert man bei 30 bis 40°C und 20 bar Wasserstoffdruck solange, bis das Reaktionsgemisch keinen Wasserstoff mehr aufnimmt.

Nach Abfiltrieren des Katalysators und Abdestillieren des Lösungmittels erhält man 225 g Rohprodukt, das gemäß HPLC Analyse circa 90 % N-Ethoxycarbonylmethylenanthranilsäure enthält.

### Beispiel 2

### Herstellung von N-Ethoxycarbonylmethylenanthranilsäure (Eintopf-Verfahren)

61 g einer 50 gew.-%igen Lösung von Glyoxylsäureethylester in Toluol (entsprechend 0,3 mol Glyoxylsäureethylester) werden mit 400 ml Toluol versetzt und 5 Stunden unter Rückfluß erhitzt, um polymere Bestandteile in monomeren Glyoxylsäureethylester zu spalten. Man kühlt danach die Lösung auf Raumtemperatur ab.
Man legt diese Glyoxylsäureethylester-Lösung, 35 g (0,25 mol) Anthranilsäure und 2,5 g eines sulfitierten Platin-Aktivkohle-Katalysator ( 5 Gew.-% Pt) in einem Autoklaven vor und hydriert unter Rühren bei 30°C und 20 bar Wasserstoffdruck 8 Stunden. Danach zeigt eine gaschromatographische Analyse (GC) eine vollständige Umsetzung der Anthranilsäure zu N-Ethoxycarbonylmethylenanthranilsäure an.
Die Suspension wird filtriert und der dabei abgetrennte Feststoff wird in 200 ml Ethanol aufgenommen und zum Rückfluß erhitzt. Die dabei anfallende Suspension wird heiß filtriert und von dem Filtrat wird das Lösungsmittel abdestilliert, bis das Wertprodukt zu kristallisieren beginnt.

Man kühlt die Mischung auf 20°C ab, filtriert und trocknet den Feststoff.
Man erhält 54 g N-Ethoxycarbonylmethylenanthranilsäure, entsprechend 80 % Ausbeute bezogen auf eingesetzte Anthranilsäure.

Die N-Ethoxycarbonylmethylenanthranilsäure wird mittels Diazomethan in den N-Ethoxycarbonylmethylenanthranilsäuremethylester umgewandelt und in dieser Form massenspektrometrisch untersucht.
MS (als Methylester nach Umsetzung mit Diazomethan)
   237 (10), 164 (30), 132 (100), 77 (25)

### Beispiel 3

### Herstellung von 4-Chlor-N-ethoxycarbonylmethylen-anthranilsäure (Eintopf-Verfahren)

61 g einer 50 gew.-%igen Lösung von Glyoxylsäureethylester in Toluol (entsprechend 0,3 mol Glyoxylsäureethylester) werden mit 400 ml Toluol versetzt und 5 Stunden unter Rückfluß erhitzt um polymere Bestandteile in monomeren Glyoxylsäureethylester zu spalten. Man kühlt danach die Lösung auf Raumtemperatur ab.

Man legt diese Glyoxylsäureethylester-Lösung, 43 g (0,25 mol) 4-Chloranthranilsäure und 2,5 g eines sulfitierten Platin-Aktivkohle-Katalysators (5 Gew.-% Pt) in einem Autoklaven vor und hydriert unter Rühren bei 30°C und 20 bar Wasserstoffdruck 8 Stunden. Danach zeigt eine gaschromatographische Analyse (GC) eine vollständige Umsetzung der 4-Chloranthranilsäure zu 4-Chlor-N-ethoxycarbonylmethylen-anthranilsäure an.

Die 4-Chlor-N-ethoxycarbonylmethylen-anthranilsäure wird mittels Diazomethan in den 4-Chlor-N-ethoxycarbonylmethylen-anthranilsäuremethylester umgewandelt und in dieser Form massenspektrometrisch untersucht.
MS (als Methylester nach Umsetzung mit Diazomethan)
   271 (15), 199 (40), 166 (100)

## Patentansprüche

1. Verfahren zur Herstellung von N-Carboxymethylenanthranilsäure-Estern der Formel (3) 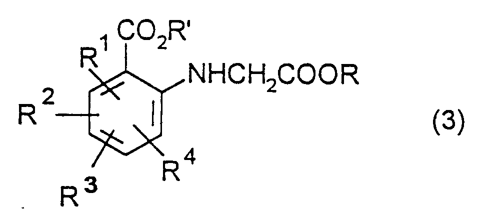 dadurch gekennzeichnet, daß man eine Verbindung der Formel (1) 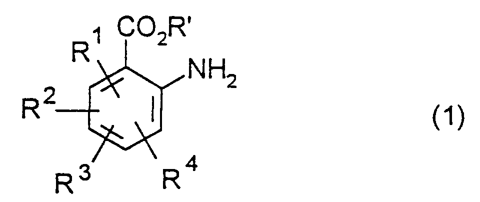 worin R' für Wasserstoff oder einen geradkettigen oder verzweigten Alkylrest mit 1 bis 4 Kohlenstoffatomen steht, R¹, R², R³, R⁴ gleich oder verschieden sind und unabhängig voneinander für Wasserstoff, Halogen, NO₂, einen geradkettigen oder verzweigten Alkyl-, Alkoxy- oder halogenierten Alkyl-Rest mit jeweils 1 bis 6 Kohlenstoffatomen stehen, mit einem Glyoxylsäureester der Formel (2)
OHC-COOR (2)
worin R für einen unsubstituierten oder einen durch Halogen, eine Alkyl-oder Alkoxygruppe mit jeweils 1 bis 4 Kohlenstoffatomen einfach oder mehrfach substituierten geradkettigen oder verzweigten Alkylrest mit 1 bis 20 Kohlenstoffatomen, Phenylrest oder Benzylrest steht, in Anwesenheit eines Lösungsmittels ausgenommen Alkohole bei 0 bis 200°C umsetzt und das Reaktionsprodukt mit Wasserstoff in Anwesenheit eines Nickel, Kobalt, Platin, Palladium, Rhodium, Ruthenium oder Verbindungen dieser Metalle enthaltenden Hydrierkatalysators bei einer Temperatur von 0 bis 200°C und einem Druck von 1 bis 150 bar reduziert.

2. Verfahren nach Anspruch 1, dadurch gekennzeichnet, daß man eine Verbindung der Formel (1), worin R' für Wasserstoff, Methyl oder Ethyl steht, einsetzt.

3. Verfahren nach Anspruch 1 oder 2, dadurch gekennzeichnet, daß man eine Verbindung der Formel (1), worin R' für Wasserstoff steht, einsetzt.

4. Verfahren nach einem oder mehreren der Ansprüche 1 bis 3, dadurch gekennzeichnet, daß man eine Verbindung der Formel (1), worin R¹, R², R³, R⁴ gleich oder verschieden sind und unabhängig voneinander für Wasserstoff, Fluor, Chlor, einen Alkyl-, Alkoxy- oder chlor- oder fluorsubstituierten Alkyl-Rest mit jeweils 1 bis 4 Kohlenstoffatomen stehen, einsetzt.

5. Verfahren nach einem oder mehreren der Ansprüche 1 bis 4, dadurch gekennzeichnet, daß man eine Verbindung der Formel (1), worin R¹, R², R³, R⁴ gleich oder verschieden sind und unabhängig voneinander für Wasserstoff, Fluor, Chlor, Methyl oder Ethyl stehen, einsetzt.

6. Verfahren nach einem oder mehreren der Ansprüche 1 bis 5, dadurch gekennzeichnet, daß man eine Verbindung der Formel (1), worin zwei oder drei der Reste R¹, R², R³, R⁴ für Wasserstoff stehen, einsetzt.

7. Verfahren nach einem oder mehreren der Ansprüche 1 bis 6, dadurch gekennzeichnet, daß man 4-Chloranthranilsäure als Verbindung der Formel (1) einsetzt.

8. Verfahren nach einem oder mehreren der Ansprüche 1 bis 7, dadurch gekennzeichnet, daß man eine Verbindung der Formel (2), worin R für einen Alkylrest mit 1 bis 6 Kohlenstoffatomen steht, einsetzt.

9. Verfahren nach einem oder mehreren der Ansprüche 1 bis 8, dadurch gekennzeichnet, daß man eine Verbindung der Formel (2), worin R für Methyl oder Ethyl steht, einsetzt.

10. Verfahren nach einem oder mehreren der Ansprüche 1 bis 9, dadurch gekennzeichnet, daß man eine Verbindung der Formel (2), worin R für Ethyl steht, einsetzt.

11. Verfahren nach einem oder mehreren der Ansprüche 1 bis 10, dadurch gekennzeichnet, daß man als Lösungsmittel einen aromatischen Kohlenwasserstoff, einem chlorierten aliphatischen oder aromatischen Kohlenwasserstoff, einen Alkylester einer aliphatischen Carbonsäure mit 1 bis 4 Kohlenstoffatomen, ein Carbonsäureamid, eine N-enthaltende heterocyclische Verbindung, einen Ether oder ein Gemisch derselben einsetzt.

12. Verfahren nach einem oder mehreren der Ansprüche 1 bis 11, dadurch gekennzeichnet, daß man als Lösungsmittel Toluol, o-Xylol, m-Xylol, p-Xylol, ein Gemisch isomerer Xylole, Ethylbenzol, Mesitylen, Chloroform, Dichlormethan, Chlorbenzol, Dichlorbenzol, Ethylacetat, Butylacetat, N,N-Dimethylacetamid, N,N-Diethylacetamid, N-Methylpyrrolidon, Tetrahydrofuran, Dioxan, Diisopropylether, Di-n-butylether oder ein Gemisch derselben einsetzt.

13. Verfahren nach einem oder mehreren der Ansprüche 1 bis 12, dadurch gekennzeichnet, daß man die Umsetzung bei 10 bis 100, insbesondere 15 bis 50°C, durchführt.

14. Verfahren nach einem oder mehreren der Ansprüche 1 bis 13, dadurch gekennzeichnet, daß man das bei der Umsetzung gebildete Wasser abtrennt.

15. Verfahren nach einem oder mehreren der Ansprüche 1 bis 14, dadurch gekennzeichnet, daß man das bei der Umsetzung gebildete Wasser mittels azeotroper Destillation, mittels eines wasserbindenden Mittels oder mittels Phasentrennung abtrennt.

16. Verfahren nach einem oder mehreren der Ansprüche 1 bis 15, dadurch gekennzeichnet, daß man bei 10 bis 100, insbesondere 15 bis 50°C, reduziert.

17. Verfahren nach einem oder mehreren der Ansprüche 1 bis 16, dadurch gekennzeichnet, daß man die Verbindung der Formel (1), die Verbindung der Formel (2), das Lösungsmittel, den Hydrierkatalysator und Wasserstoff zusammen vorlegt und die Umsetzung und die Reduktion zugleich durchführt.

## Claims

1. A process for the preparation of an N-carboxymethyleneanthranilic acid ester of the formula (3) 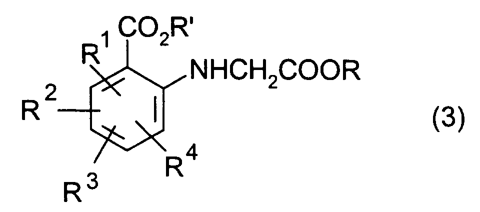 which comprises reacting a compound of the formula (1) 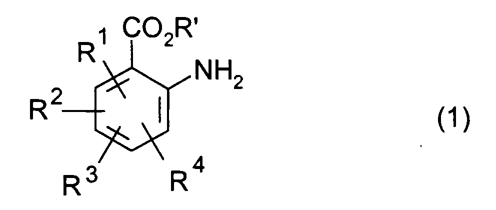 in which R' is hydrogen or a straight-chain or branched alkyl radical having 1 to 4 carbon atoms and R¹, R², R³ and R⁴ are identical or different and independently of one another are hydrogen, halogen, NO₂, a straight-chain or branched alkyl, alkoxy or halogenated alkyl radical having in each case 1 to 6 carbon atoms, with a glyoxylic acid ester of the formula (2)
OHC-COOR (2)
in which R is a straight-chain or branched alkyl radical having 1 to 20 carbon atoms, phenyl radical or benzyl radical, which are unsubstituted or mono- or polysubstituted by halogen or an alkyl or alkoxy group having in each case 1 to 4 carbon atoms, in the presence of a solvent -excluding alcohols- at 0 to 200°C, and reducing the reaction product with hydrogen in the presence of a hydrogenation catalyst comprising nickel, cobalt, platinum, palladium, rhoduim, ruthenium or compounds of these metals at a temperature of 0 to 200°C under a pressure of 1 to 150 bar.

2. The process as claimed in claim 1, wherein a compound of the formula (1) in which R' is hydrogen, methyl or ethyl is employed.

3. The process as claimed in claim 1 or 2, wherein a compound of the formula (1) in which R' is hydrogen is employed.

4. The process as claimed in one or more of claims 1 to 3, wherein a compound of the formula (1) in which R¹, R², R³ and R⁴ are identical or different and independently of one another are hydrogen, fluorine, chlorine or an alkyl, alkoxy or chlorine- or fluorine-substituted alkyl radical having in each case 1 to 4 carbon atoms is employed.

5. The process as claimed in one or more of claims 1 to 4, wherein a compound of the formula (1) in which R¹, R², R³ and R⁴ are identical or different and independently of one another are hydrogen, fluorine, chlorine, methyl or ethyl is employed.

6. The process as claimed in one or more of claims 1 to 5, wherein a compound of the formula (1) in which two or three of the radicals R¹, R², R³ and R⁴ are hydrogen is employed.

7. The process as claimed in one or more of claims 1 to 6, wherein 4-chloroanthranilic acid is employed as the compound of the formula (1).

8. The process as claimed in one or more of claims 1 to 7, wherein a compound of the formula (2) in which R is an alkyl radical having 1 to 6 carbon atoms is employed.

9. The process as claimed in one or more of claims 1 to 8, wherein a compound of the formula (2) in which R is methyl or ethyl is employed.

10. The process as claimed in one or more of claims 1 to 9, wherein a compound of the formula (2) in which R is ethyl is employed.

11. The process as claimed in one or more of claims 1 to 10, wherein an aromatic hydrocarbon, a chlorinated aliphatic or aromatic hydrocarbon, an alkyl ester of an aliphatic carboxylic acid having 1 to 4 carbon atoms, a carboxylic acid amide, an N-containing heterocyclic compound, an ether or a mixture thereof is employed as the solvent.

12. The process as claimed in one or more of claims 1 to 11, wherein toluene, o-xylene, m-xylene, p-xylene, a mixture of isomeric xylenes, ethylbenzene, mesitylene, chloroform, methylene chloride, chlorobenzene, dichlorobenzene, ethyl acetate, butyl acetate, N,N-dimethylacetamide, N,N-diethylacetamide, N-methylpyrrolidone, tetrahydrofuran, dioxane, diisopropyl ether, di-n-butyl ether or a mixture thereof is employed as the solvent.

13. The process as claimed in one or more of claims 1 to 12, wherein the reaction is carried out at 10 to 100, in particular 15 to 50°C.

14. The process as claimed in one or more of claims 1 to 13, wherein the water formed during the reaction is separated off.

15. The process as claimed in one or more of claims 1 to 14, wherein the water formed during the reaction is separated off by means of azeotropic distillation, by means of a water-binding agent or by means of phase separation.

16. The process as claimed in one or more of claims 1 to 15, wherein the reduction is carried out at 10 to 100, in particular 15 to 50°C.

17. The process as claimed in one or more of claims 1 to 16, wherein the compound of the formula (1), the compound of the formula (2), the solvent, the hydrogenation catalyst and hydrogen are introduced into the reaction vessel together and the reaction and the reduction are carried out at the same time.

## Revendications

1. Procédé de préparation des esters de l'acide N-carboxyméthylène anthranilique de formule (3) 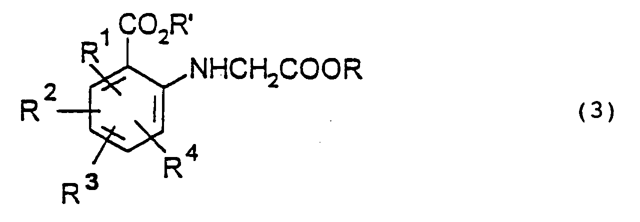 caractérisé en ce que l'on fait réagir un composé de formule (1) 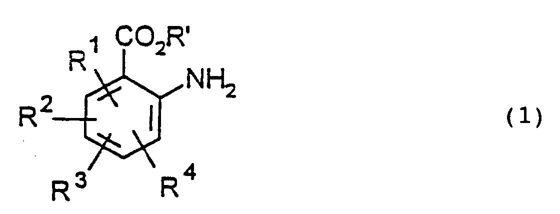 dans laquelle R' représente un atome d'hydrogène ou un reste alkyle à chaîne droite ou ramifiée comportant 1 à 4 atomes de carbone, R¹, R², R³, R⁴ sont identiques ou différents et représentent, indépendamment les uns des autres, des atomes d'hydrogène, des atomes d'halogènes, NO₂, un reste alkyle, un reste alkoxy ou un reste alkyle halogéné comportant chacun 1 à 6 atomes de carbone, à chaîne droite ou ramifiée, avec un ester de l'acide glyoxylique de formule (2)
OHC-CCOR (2)
où R représente un reste benzyle, un reste phényle ou un reste alkyle comportant 1 à 20 atomes de carbone, à chaîne droite ou ramifiée, non substitué ou substitué une ou plusieurs fois par des atomes d'halogènes, un groupes alkyle ou alkoxy comportant chacun 1 à 4 atomes de carbone, en présence d'un solvant - à l'exception des alcools - à une température de 20 à 200°C et on réduit le produit réactionnel par l'hydrogène en présence d'un catalyseur d'hydrogénation au nickel, au cobalt, au platine, au palladium, au rhodium, au ruthénium ou aux composés de ces métaux, à une température de 0 à 200°C et une pression de 1 à 150 bars.

2. Procédé selon la revendication 1, caractérisé en ce que l'on emploie un composé de formule (1) où R' représente un atome d'hydrogène, des groupes méthyle ou éthyle.

3. Procédé selon la revendication 1 ou 2, caractérisé en ce que l'on emploie un composé de formule (1) où R' représente un atome d'hydrogène.

4. Procédé selon une ou plusieurs des revendications 1 à 3, caractérisé en ce que l'on emploie un composé de formule (1) où R¹, R², R³, R⁴ sont identiques ou différents et représentent, indépendamment les uns des autres, des atomes d'hydrogène, de fluor, de chlore, un reste alkyle présentant 1 à 4 atomes de carbone substitué par des substituants alkyle, alkoxy ou chlore ou fluor.

5. Procédé selon une ou plusieurs des revendications 1 à 4, caractérisé en ce que l'on emploie un composé de formule (1) où R¹, R², R³, R⁴ sont identiques ou différents et représentent, indépendamment les uns des autres, des atomes d'hydrogène, de fluor, de chlore, des groupes méthyle ou éthyle.

6. Procédé selon une ou plusieurs des revendications 1 à 5, caractérisé en ce que l'on emploie un composé de formule (1) où deux ou trois des restes R¹, R², R³, R⁴ représentent des atomes d'hydrogène.

7. Procédé selon une ou plusieurs des revendications 1 à 6, caractérisé en ce que l'on emploie l'acide 4-chloranthranilique en tant que composé de formule (1).

8. Procédé selon une ou plusieurs des revendications 1 à 7, caractérisé en ce que l'on emploie un composé de formule (2) où R représente un reste alkyle comportant 1 à 6 atomes de carbone.

9. Procédé selon une ou plusieurs des revendications 1 à 8, caractérisé en ce que l'on emploie un composé de formule (2) où R représente des groupes méthyle ou éthyle.

10. Procédé selon une ou plusieurs des revendications 1 à 9, caractérisé en ce que l'on emploie un composé de formule (2) où R représente un groupe éthyle.

11. Procédé selon une ou plusieurs des revendications 1 à 10, caractérisé en ce que l'on emploie en tant que solvant un hydrocarbure aromatique, un hydrocarbure aliphatique ou aromatique chloré, un ester alkylique d'un acide carboxylique aliphatique comportant 1 à 4 atomes de carbone, un amide d'acide carboxylique, un composé hétérocyclique azoté, un éther, ou un mélange de ces composés.

12. Procédé selon une ou plusieurs des revendications 1 à 11, caractérisé en ce que l'on emploie en tant que solvant le toluène, l'o-xylène, le m-xylène, le p-xylène, un mélange de xylènes isomères, l'éthylbenzène, le mésitylène, le chloroforme, le dichlorométhane, le chlorobenzène, le dichlorobenzène, l'acétate d'éthyle, l'acétate de butyle, le N,N-diméthylacétamide, le N,N-diéthylacétamide, la N-méthylpyrrolidone, le tétrahydrofuranne, la dioxanne, l'éther diisopropylique, l'éther di-n-butylique ou un mélange de ces composés.

13. Procédé selon une ou plusieurs des revendications 1 à 12, caractérisé en ce que l'on met en oeuvre la réaction à une température de 10 à 100, en particulier de 15 à 50°C.

14. Procédé selon une ou plusieurs des revendications 1 à 13, caractérisé en ce que l'on élimine l'eau formée au cours de la réaction.

15. Procédé selon une ou plusieurs des revendications 1 à 14, caractérisé en ce que l'on élimine par distillation azéotropique l'eau formée au cours de la réaction, à l'aide d'un agent fixant l'eau ou par séparation de phases.

16. Procédé selon une ou plusieurs des revendications 1 à 15, caractérisé en ce que l'on réalise la réduction à une température de 10 à 100, en particulier de 15 à 50°C.

17. Procédé selon une ou plusieurs des revendications 1 à 16, caractérisé en ce que l'on place ensemble le composé de formule (1), le composé de formule (2), le solvant, le catalyseur d'hydrogénation et l'hydrogène et l'on met en oeuvre la réaction et la réduction simultanément.
